# EUROPEAN PATENT SPECIFICATION

(11) **EP 1 660 483 B1**
(45) Date of publication and mention of the grant of the patent: **24.12.2008**
(21) Application number: 04764687.2
(22) Date of filing: 26.08.2004
(51) Int. Cl.: C07D 401/12, A61K 31/47

(54) **8-(1-PIPERAZINYL)-QUINOLINE DERIVATIVES AND THEIR USE IN THE TREATMENT OF CNS DISORDERS**
8-(1-PIPERAZINYL)-CHINOLINDERIVATE UND DEREN VERWENDUNG BEI DER BEHANDLUNG VON ZNS-ERKRANKUNGEN
DERIVES DE 8- (1-PIPERAZINYL)- QUINOLEINE ET LEUR UTILISATION POUR LE TRAITEMENT DE TROUBLES DU SYSTEME NERVEUX CENTRAL

(30) Priority: 29.08.2003 GB 0320320
(43) Date of publication of application: 31.05.2006
(73) Proprietor: GLAXO GROUP LIMITED, Greenford, Middlesex UB6 ONN (GB)
(72) Inventor: JOHNSON, Christopher Norbert, GlaxoSmithKline, Harlow Essex CM19 5AW (GB); MOSS, Stephen Frederick, GlaxoSmithKline, Harlow Essex CM19 5AW (GB); TAIT, Malcolm M., GlaxoSmithKline, Harlow Essex CM19 5AW (GB); WITTY, David, R., GlaxoSmithKline, Harlow Essex CM19 5AW (GB)
(74) Representative: Thornley, Rachel Mary
(86) International application number: PCT/EP2004/009724
(87) International publication number: WO 2005/021530

(56) References cited:
- WO-A-01/32646
- WO-A-03/042208
- WO-A-03/080580
- WO-A-03/080608
- US-A1- 2003 158 202
- US-B1- 6 423 717

## Description

This invention relates to novel quinoline compounds having pharmacological activity, to processes for their preparation, to compositions containing them and to their use in the treatment of CNS and other disorders.

JP 02262627 (Japan Synthetic Rubber Co) describes a series of substituted quinoline derivatives useful as wavelength converting elements. WO 00/42026 (Novo Nordisk) describes a series of quinoline and quinoxaline compounds for use as GLP-1 agonists.

A structurally novel class of compounds has now been found which also possess affinity for the 5-HT₆ receptor. The present invention therefore provides, in a first aspect, a compound of formula (I) or a pharmaceutically acceptable salt thereof: 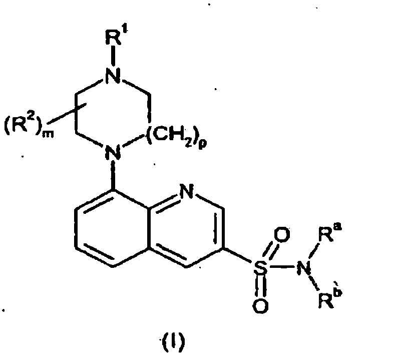 wherein:
R¹ represents hydrogen or methyl;
   wherein said alkyl, cycloalkyl, aryl, heteroaryl or heterocyclyl groups of R¹ may be optionally substituted by one or more (eg. 1, 2 or 3) halogen, C₁₋₆ alkyl, C₁₋₆ alkoxy, cyano, amino or trifluoromethyl groups;
R² represents hydrogen or C₁₋₆ alkyl;
m represents an integer from 1 to 4, such that when m is an integer greater than two
R² groups may instead be linked to form a CH₂, (CH₂)₂ or (CH₂)₃ group;
   when R¹ represents methyl, R¹ may optionally be linked to R² to form a group (CH₂)₂, (CH₂)₃ or (CH₂)₄;
p represents 1 or 2;
R^{a} and R^{b} together with the nitrogen atom to which they are attached represent a nitrogen containing heterocyclyl group optionally substituted by a halogen atom;
   wherein said nitrogen containing heterocyclyl represents a 4-7 membered monocyclic saturated or partially unsaturated aliphatic ring or a 4-7 membered monocyclic saturated or partially unsaturated aliphatic ring containing 1 to 3 heteroatoms selected from oxygen and nitrogen fused to a benzene or monocyclic heteroaryl ring;
or solvates thereof.

Alkyl groups, whether alone or as part of another group, may be straight chain or branched and the groups alkoxy and alkanoyl shall be interpreted similarly. Alkyl moieties are more preferably C₁₋₄ alkyl, eg. methyl or ethyl. The term 'halogen' is used herein to describe, unless otherwise stated, a group selected from fluorine, chlorine, bromine or iodine.

The term "heteroaryl" is intended to mean a 5-7 membered monocyclic aromatic or a fused 8-10 membered bicyclic aromatic ring containing 1 to 3 heteroatoms selected from oxygen, nitrogen and sulfur. Suitable examples of such monocyclic aromatic rings include thienyl, furyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused aromatic rings include benzofused aromatic rings such as quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl, benzothiadiazolyl and the like. Heteroaryl groups, as described above, may be linked to the remainder cf the molecule via a carbon atom or, when present, a suitable nitrogen atom except where otherwise indicated above.

The term "heterocyclyl" is intended to mean a 4-7 membered monocyclic saturated or partially unsaturated aliphatic ring or a 4-7 membered monocyclic saturated or partially unsaturated aliphatic ring containing 1 to 3 heteroatoms selected from oxygen or nitrogen fused to a benzene or monocyclic heteroaryl ring. Suitable examples of such monocyclic rings include pyrrolidinyl, piperidinyl, piperazinyl, morpholinyl, thiamorpholinyl, diazepanyl, azepanyl, dihydroimidazolyl, tetrahydropyranyl, tetrahydrothiapyranyl and tetrahydrofuranyl. Suitable examples of benzofused heterocyclic rings include dihydroindolyl, dihydroisoindolyl, tetrahydroquinolinyl, tetrahydrobenzazepinyl and tetrahydroisoquinolinyl.

The term "nitrogen containing heterocyclyl" is intended to represent any heterocyclyl group as defined above which contains a nitrogen atom.

Preferably, R¹ represents hydrogen. Preferably R² represents hydrogen, methyl (eg. 3-methyl, 2-methyl, 3,3-dimethyl or 2,5-dimethyl) or is linked to R¹ to form a (CH₂)₃ group.
Preferably, m and p independently represent 1 or 2, more preferably m and p both represent 1. More preferably R² represents hydrogen or methyl (e.g. 3-methyl), especially hydrogen. In one preferred embodiment, m represents 2 and both R² groups are linked to form a CH₂ group linking C-2 and C-5 of the piperazine ring.

Preferably, R^{a} and R^{b} together with the nitrogen atom to which they are attached form a nitrogen containing heterocyclyl group (eg. 1-piperidinyl, 4-morpholinyl, 1-(2,3-dihydro-1*H*-indolyl) or 2-(2,3-dihydro-1*H*-isoindolyl)) optionally substituted by a halogen atom (eg. fluorine). More preferably, R^{a} and R^{b} together with the nitrogen atom to which they are attached form 1-(2,3-dihydro-1*H*-indolyl).

Preferred compounds according to the invention include examples E1-E5 as shown below, or a pharmaceutically acceptable salt thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid. The present invention includes within its scope all possible stoichiometric and non-stoichiometric forms.

The compounds of formula (I) may be prepared in crystalline or non-crystalline form, and, if crystalline, may optionally be solvated, eg. as the hydrate. This invention includes within its scope stoichiometric solvates (eg. hydrates) as well as compounds containing variable amounts of solvent (eg. water).

Certain compounds of formula (I) are capable of existing in stereoisomeric forms e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention also provides a process for the preparation of a compound of formula (I) or a pharmaceutically acceptable salt thereof, which process comprises:
(a) reacting a compound of formula (II) 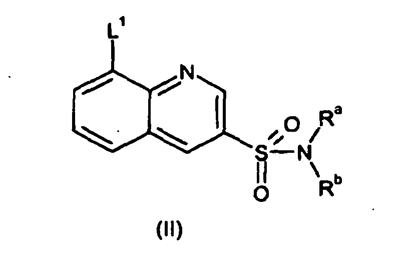 with a compound of formula (III) 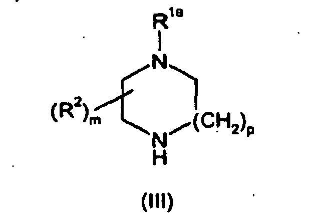 wherein R^{1a} is as defined for R¹ or an *N*-protecting group, R², R^{a}, R^{b}, m and p are as defined above and L¹ represents a suitable leaving group, such as a halogen atom (e.g. a bromine or iodine atom) or a trifluoromethylsulfonyloxy group, and thereafter as necessary removing an R^{1a} *N*-protecting group. The *N*-protecting group used may be any conventional group e.g. *t*-butyloxycarbonyl (Boc) or benzyloxycarbonyl. Further *N-*protecting groups which may be used include methyl; or
(b) reacting a compound of formula (IV) 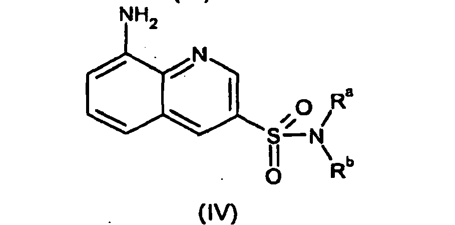 with a compound of formula (V) 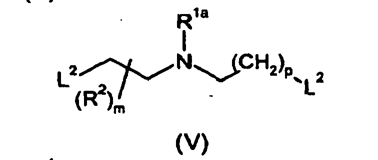 wherein R^{1a} is as defined for R¹ or an N-protecting group, R², R^{a}, R^{b}, m and p are as defined above, and L² represents a suitable leaving group, such as a halogen atom and thereafter as necessary removing an R^{1a} N-protecting group;
(c) deprotecting a compound of formula (I) which is protected; and thereafter optionally
(d) interconversion to other compounds of formula (I) and/or forming a pharmaceutically acceptable salt and/or solvate.

Process (a) may be performed in the presence of a palladium, nickel or copper catalyst, for example a mixture of a palladium source such as Pd₂(dba)₃ and a suitable ligand such as (R)-, (S)- or (±)-2,2'-bis(diphenylphosphino)-1,1'-binaphthyl (BINAP) or (2-dicyclohexylphosphanylphenyl)-dimethylamine or 1,1'-bis-diphenylphosphinoferrocene, together with a suitable base such as sodium *t*-butoxide, in an inert solvent such as 1,4-dioxane.

Process (b) may be performed in the presence of a suitable base, such as sodium carbonate using a suitable solvent such as n-butanol.

In processes (a), (b) and (c) examples of protecting groups and the means for their removal can be found in T. W. Greene 'Protective Groups in Organic Synthesis' (J. Wiley and Sons, 1991). Suitable amine protecting groups include sulphonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an acid such as hydrochloric acid) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃) which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid. A further amine protecting group includes methyl which may be removed using standard methods for N-dealkylation (e.g. 1-chloroethyl chloroformate under basic conditions followed by treatment with methanol).

Process (d) may be performed using conventional interconversion procedures such as epimerisation, oxidation, reduction, reductive alkylation, alkylation, nucleophilic or electrophilic aromatic substitution, ester hydrolysis or amide bond formation. For example, *N*-dealkylation of a compound of formula (I) wherein R¹ represents a methyl group to give a compound of formula (I) wherein R¹ represents hydrogen. It will be appreciated that such interconversion may be interconversion of protected derivatives of formula (I) which may subsequently be deprotected following interconversion.

In addition, process (d) may comprise, for example, reacting a compound of formula (I), wherein R¹ represents hydrogen, with an aldehyde or ketone in the presence of a reducing agent in order to generate a compound of formula (I) where R¹ represents methyl. This may be performed using a hydride donor agent such as sodium cyanoborohydride, sodium triacetoxyborohydride or a resin bound form of cyanoborohydride in an alcoholic solvent such as ethanol and in the presence of an acid such as acetic acid, or under conditions of catalytic hydrogenation. Alternatively, such a transformation may be carried out by reacting a compound of formula (I), wherein R¹ represents hydrogen, with a compound of formula R¹-L, wherein R¹ represents methyl and L represents a leaving group such as a halogen atom (e.g. bromine or iodine) or methylsulfonyloxy group, optionally in the presence of a suitable base such as potassium carbonate, sodium hydride or triethylamine using an appropriate solvent such as *N,N*-dimethylformamide, tetrahydrofuran or a C₁₋₄alkanol.

Compounds of formula (II) may be prepared by reacting a compound of formula (VI) 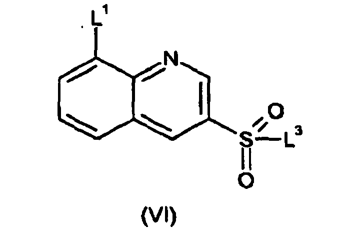 wherein L¹ is as defined above and L³ represents a suitable leaving group such as halogen, e.g. fluorine or chlorine; with a compound of formula NHR^{a}R^{b} wherein R^{a} and R^{b} are as defined above. Such a reaction may advantageously carried out in an inert solvent such as dichloromethane in the presence of a base such as triethylamine or an excess of the compound of formula NHR^{a}R^{b}.

Compounds of formula (VI) may be prepared in accordance with the following scheme: 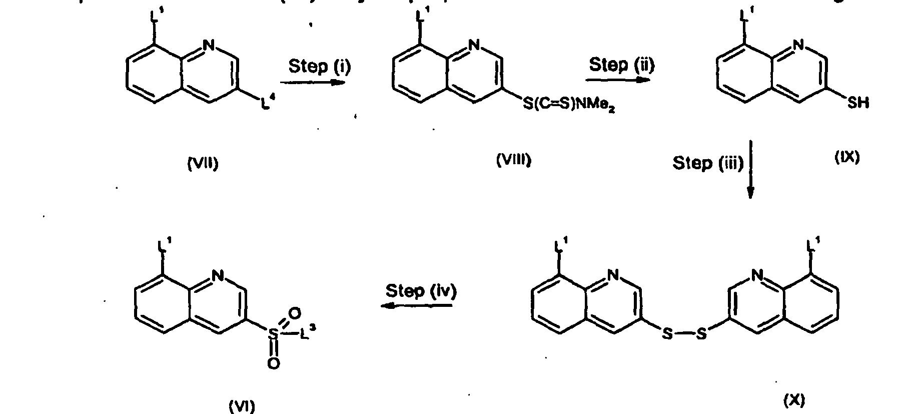 wherein L¹ and L³ are as defined above and L⁴ represents a suitable leaving group such as halogen (e.g. bromine or iodine), preferably L¹ and L⁴ represent different leaving groups (e.g. L¹ and L⁴ represent chlorine and iodine, respectively).

Step (i) typically comprises addition of a sulfur nucleophile using a suitable metal salt of a dialkyldithiocarbamic acid (e.g. zinc dimethyldithiocarbamate) in the presence of a suitable copper (I) salt (e.g. copper triflate) and a suitable ligand such as 1,2-dimethylaminoethane in an appropriate solvent such as dimethylsulfoxide at an elevated temperature (e.g. 90 °C).

Step (ii) typically comprises cleavage of the thiocarbamoyl moiety using a suitable nucleophile, such as sodium sulfide in an appropriate solvent, such as aqueous methanol.

Step (iii) typically comprises oxidation to the disulfide using an oxidant, such as iodine and such a process may be advantageously carried out in a biphasic reaction medium, such as aqueous potassium monohydrogenphosphate and dichloromethane.

Step (iv) typically comprises an oxidation with concomitant introduction of the group L³. When L³ represents a chlorine atom this process may be advantageously carried out using sulfuryl chloride in the presence of an oxidant, such as potassium nitrate in an inert solvent, such as acetonitrile.

Compounds of formula (VII) where L⁴ represents halogen may be prepared by reaction of a compound of formula (XI) 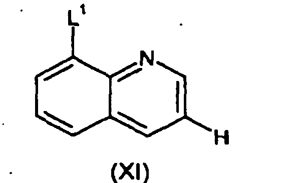 wherein L¹ is as defined above, with an appropriate halogenating reagent. For example, when L⁴ represents an iodine atom, an appropriate process comprises reaction of a compound of formula (XI) with N-iodosuccinimide in the presence of acetic acid at elevated temperature (e.g. 80 °C). Such a reaction may be advantageously carried out using acetic acid as solvent.

Compounds of formula (IV) may be prepared in accordance with the following scheme: 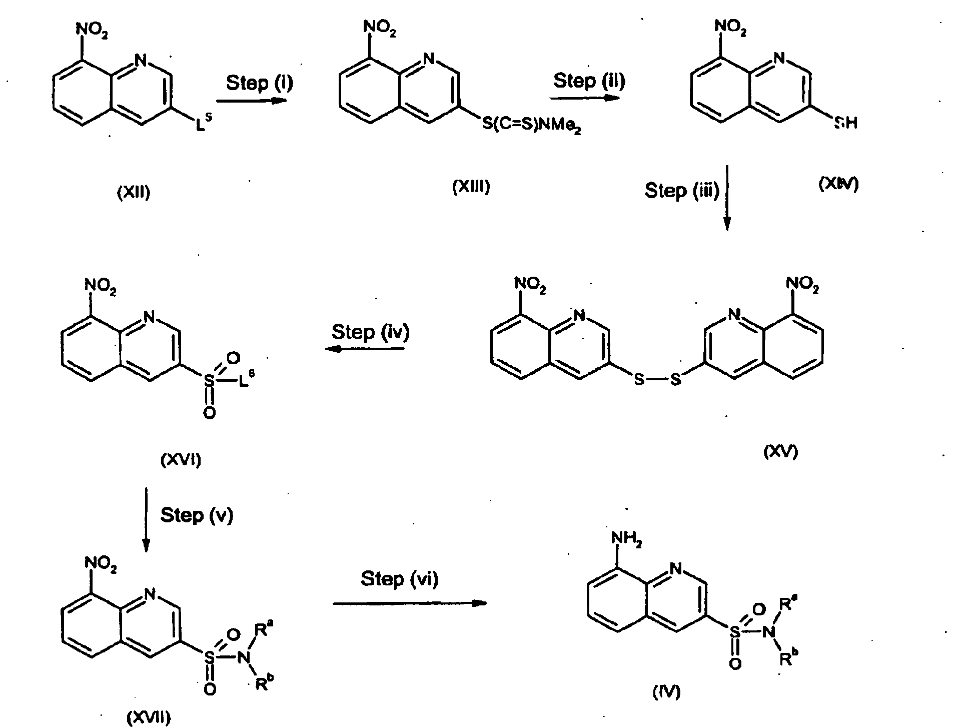 wherein R^{a} and R^{b} are as defined above, L⁵ represents a suitable leaving group such as a halogen atom (eg. bromine or iodine) and L⁶ represents a suitable leaving group such as a halogen atom (eg. fluorine or chlorine).

Step (i) typically comprises addition of a sulfur nucleophile using a suitable metal salt of a dialkyldithiocarbamic acid (e.g. zinc dimethyldithiocarbamate) in the presence of a suitable copper (I) salt (e.g. copper triflate) and a suitable ligand such as 1,2-dimethylaminoethane in an appropriate solvent such as dimethylsulfoxide at an elevated temperature (e.g. 90 °C).

Step (ii) typically comprises cleavage of the thiocarbamoyl moiety using a suitable nucleophile, such as sodium sulfide or trimethylsilanol sodium salt, in an appropriate solvent such as aqueous methanol or dimethyl sulfoxide respectively.

Step (iii) typically comprises oxidation to the disulfide using an oxidant, such as iodine and such a process may be advantageously carried out in a biphasic reaction medium, such as aqueous potassium monohydrogenphosphate and dichloromethane.

Step (iv) typically comprises an oxidation with concomitant introduction of the group L⁶. When L⁶ represents a chlorine atom this process may be advantageously carried out using sulfuryl chloride in the presence of an oxidant, such as potassium nitrate in an inert solvent, such as acetonitrile.

Step (v) typically comprises addition of a compound of formula NHR^{a}R^{b} wherein R^{a} and R^{b} are as defined above, using a procedure analogous to that used to prepare compounds of formula (II) from compounds of formula (VI).

Step (vi) typically comprises reduction of the nitro group using a suitable reducing agent, e.g. iron powder in acetic acid or aqueous titanium trichloride in appropriate organic solvent such as tetrahydrofuran.

An alternative process for the preparation of compounds of formula (II) as defined above where L¹ represents halogen comprises diazotisation of a compound of formula (IV) as defined above using standard methods (e.g. use of sodium nitrite and an appropriate acid) followed by treatment of the resulting diazonium salt with an appropriate reagent for the introduction of the halogen (e.g. a copper (I) halide such as copper (I) bromide, or potassium iodide).

Compounds of formula (III), (V), (XI) and (XII) are known in the literature or can be prepared by analogous methods.

Pharmaceutically acceptable salts may be prepared conventionally by reaction with the appropriate acid or acid derivative.

Compounds of formula (I) and their pharmaceutically acceptable salts have affinity for the 5-HT₆ receptor and are believed to be of potential use in the treatment of certain CNS disorders such as anxiety, depression, epilepsy, obsessive compulsive disorders, migraine, cognitive memory disorders (e.g. Alzheimers disease, age related cognitive decline and mild cognitive impainnent), Parkinsons Disease, ADHD (Attention Deficit Disorder/Hyperactivity Syndrome), sleep disorders (including disturbances of Circadian rhythm), feeding disorders such as anorexia and bulimia, panic attacks, withdrawal from drug abuse such as cocaine, ethanol, nicotine and benzodiazepines, schizophrenia (in particular cognitive deficits of schizophrenia), stroke and also disorders associated with spinal trauma and/or head injury such as hydrocephalus. Compounds of the invention are also expected to be of use in the treatment of certain Gl (gastrointestinal) disorders such as IBS (Irritable Bowel Syndrome). Compounds of the invention are also expected to be of use in the treatment of obesity.

Thus the invention also provides a compound of formula (I) or a pharmaceutically acceptable salt thereof, for use as a therapeutic substance, in particular in the treatment or prophylaxis of the above disorders. In particular the invention provides for a compound of formula (I) or a pharmaceutically acceptable salt thereof, for use in the treatment of depression, anxiety. Alzheimers disease, age related cognitive decline, ADHD, obesity, mild cognitive impairment, schizophrenia, cognitive deficits in schizophrenia and stroke.

A method of treatment or prophylaxis of the above disorders, in mammals including humans, is also provided which comprises administering to the sufferer a therapeutically effective amount of a compound of formula (I) or a pharmaceutically acceptable salt thereof.

In another aspect, the invention provides the use of a compound of formula (I) or a pharmaceutically acceptable salt thereof in the manufacture of a medicament for use in the treatment or prophylaxis of the above disorders.

5-HT₆ antagonists have the potential to be capable of increasing basal and learning-Induced polysialylated neuron cell frequency in brain regions such as the rat medial temporal lobe and associated hippocampus, as described in WO 03/066056. Thus, according to a further aspect of the present invention, we provide a method of promoting neuronal growth within the central nervous system of a mammal which comprises the step of administering a compound of formula (I) or a pharmaceutically acceptable salt thereof.

In order to use the compounds of formula (I) in therapy, they will normally be formulated into a pharmaceutical composition in accordance with standard pharmaceutical practice.

The present invention also provides a pharmaceutical composition, which comprises a compound of formula (I) or a pharmaceutically acceptable salt thereof, and a pharmaceutically acceptable carrier.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 200 mg, for example 20 to 40 mg; and such unit doses will preferably be administered once a day, although administration more than once a day may be required; and such therapy may extend for a number of weeks or months.

All publications, including but not limited to patents and patent applications, cited in this specification are herein incorporated by reference as if each individual publication were specifically and individually indicated to be incorporated by reference herein as though fully set forth.

The following Descriptions and Examples illustrate the preparation of compounds of the invention.

### Description 1

### 8-Chloro-3-iodoquinoline (D1)

*N*-Iodosuccinimide (67.9 g, 0.30 mmol) was added in portions to a stirred solution of 8-chloroquinoline (49 g, 0.30 mmol) (J. Org. Chem., 1987, 52, 1673-80) in acetic acid (300 ml) at 70 °C under argon. The mixture was heated to 70 °C for 18 h and then concentrated *in vacuo.* The residue was redissolved in dichloromethane (600 ml) and the solution was washed successively with 10% aqueous sodium thiosulfate solution (2 x 300 ml) and 10% aqueous sodium hydrogen carbonate solution (2 x 300 ml), dried (MgSO₄) and concentrated *in vacuo* to a solid. The solid was recrystallised from ethyl acetate to afford the title compound (D1) as a yellow solid (42 g, 0.145 mol, 48%). The residue from recrystallisation was purified by chromatography over silica gel eluting.with a toluene/acetone gradient to afford a second crop of the product (18 g, total yield 69%). δ_{H} (CDCl₃) 7.49 (1H, t, J = 8.1 Hz), 7.65 (1 H, dd, J = 1.4Hz, 8.3Hz), 7.85 (1H, dd, J = 1.3Hz, 7.4Hz), 8.57 (1 H, d, J = 2.1Hz), 9.15 (1 H, d J = 2.1 Hz).
Mass Spectrum: C₉H₅CIIN requires 289, 291; found 290, 292 (MH⁺)

### Description 2

### 8-Chloro-3-quinolinyl dimethyldithiocarbamate (D2)

*N, N'*-Dimethylethylene diamine (0.15 g, 1.7 mmol) was added to a stirred mixture of dimethyldithiocarbamic acid zinc salt (5.8 g, 19.0 mmol), copper (I) triflate (0.44 g, 0.9 mmol) and 8-chloro-3-iodoquinoline (D1) (5 g, 19.0 mmol) in dimethyl sulfoxide (25 ml). This mixture was heated to 90 °C for 3 h, then cooled to ambient temperature, diluted with dichloromethane (100 ml), stirred with activated charcoal (1 g) and filtered. The filtrate was washed with water (2 x 200 ml), dried (MgSO₄) and concentrated *in vacuo* to give the title compound (D2) as a solid in crude form (5.3 g, 19 mmol, 100%) which was used directly in the next stage (see D3).
δ_{H} (CDCl₃) 3.57 (6H, s), 7.52 (1H, t, J = 7.8Hz), 7.76 (1H, dd, J = 1.3Hz, 8.2Hz), 7.90 (1H, dd, J = 1.3Hz, 7.5Hz), 8.28 (1 H, d, J = 2.1 Hz), 8.95 (1 H, d, J = 2.1 Hz).
Mass Spectrum: C₁₂H₁₁ClN₂S₂ requires 282, 284; found 283, 285 (MH⁺)

### Description 3

### 8-Chloro-3-quinolinethiol (D3)

A solution of sodium sulfide (1.27 g, 5.3 mmol) in water (7 ml) was added to a stirred suspension of 8-chloro-3-quinolinyl dimethyldithiocarbamate (D2) (0.5 g, 1.8 mmol) in methanol (7 ml) and 1,4-dioxan (7 ml) at ambient temperature under argon. The suspension was heated at 60 °C for 18 h then at 80 °C for 1 h. The reaction mixture was cooled and poured into a 5% aqueous solution of citric acid (100 ml) and the whole was extracted with dichloromethane (3 x 100 ml). The combined organic extracts were dried (MgSO₄) and concentrated *in vacuo* to afford the title compound (D3) in crude form as a solid (0.24 g, 1.2 mmol, 70%) which was used directly in the next stage (see D4). Mass Spectrum: C₉H₆ClNS requires 195, 197; found 196, 198 (MH⁺)

### Description 4

### 3,3'-Dithiobis(8-chloroquinoline) (D4)

A 10% aqueous solution of potassium hydrogen phosphate (50 ml) and a solution of 8-chloro-3-quinolinethiol (D3) (3.2 g, 16.1 mmol) in dichloromethane (50 ml) was treated with iodine (4.1 g, 16.1 mmol). The mixture was vigorously stirred at ambient temperature for 1.5 h, then was diluted with dichloromethane (300 ml), vigorously shaken and the layers separated. The organic extract was dried (MgSO₄) and concentrated to a solid residue. A solution of this residue in ethyl acetate was pre-adsorbed onto silica gel and purified by chromatography on silica gel eluting with an ethyl acetate/hexane gradient to afford the title compound (D4) as a solid (2.1 g, 5.3 mmol, 67%).
δ_{H} (CDCl₃) 7.48 (1H, t, J = 8.2Hz), 7.65 (1 H, dd, J = 1.3Hz, 8.3Hz), 7.83 (1 H, dd, J = 1.3Hz, 7.5Hz), 8.26 (1 H, d, J = 2.3Hz), 9.11 (1 H, d, J = 2.3Hz).
Mass Spectrum: C₁₈H₁₀Cl₂N₂S₂ requires 388, 390; found 389, 391 (MH⁺)

### Description 5

### 8-Chloro-3-quinolinesulfonyl chloride (D5)

Potassium nitrate (2.46 g, 24.3 mmol) was added portionwise to a stirred suspension of the 3,3'-dithiobis(8-chloroquinoline) (D4) (1.9 g, 4.9 mmol) in acetonitrile (50 ml) at 0 - 5 °C. To this mixture was then added sulfuryl chloride (2.3 ml, 24.3 mmol) dropwise over 0.3 h and the temperature was maintained for 1 h. To the cold reaction mixture was then added a cold saturated aqueous solution of sodium carbonate (45 ml) and the whole was quickly extracted with diethyl ether (3 x 100 ml). The combined extracts were dried (MgSO₄) and concentrated *in vacuo* to give the title compound (D5) as a solid (1.3 g, 5.0 mmol, 51%).
δ_{H} (CDCl₃) 7.71 (1H, t, J = 8.0Hz), 8.00 (1H, dd, J = 1.3Hz, 8.2Hz), 8.11 (1H, dd, J = 1.3Hz, 7.5Hz), 8.90 (1H, d, J = 2.3Hz), 9.51 (1 H, d, J = 2.3Hz).
Mass Spectrum: C₉H₅Cl₂NO₂S requires 261, 263; found 262, 264 (MH⁺)

### Description 6

### 8-Chloro-3-(2,3-dihydro-1H-indol-1-ylsulfonyl)quinoline (D6)

To a stirred solution of 8-chloro-3-quinolinesulfonyl chloride (D5) (0.63 g, 2.4 mmol) in dichloromethane (10 ml) was added 2,3-dihydro-1*H*-indole (0.32 g, 2.6 mmol) and triethylamine (0.37 ml, 2.6 mmol) at 0 - 5°C under argon. After 1 h at this temperature range, the mixture was warmed to ambient temperature and stirred for 18 h. The reaction mixture was washed with water (2 x 15 ml), dried (MgSO₄) and concentrated *in vacuo* to an oil. The oil was purified by chromatography over silica gel eluting with a gradient of ethyl acetate/hexane to afford the title compound (D6) as a solid (0.62 g, 1.8 mmol, 75%).
δ_{H} (CDCl₃) 2.93 (2H, t, J = 8.4Hz), 4.02 (2H, t, J = 8.4Hz), 6.96-7.09 (2H, m), 7.19-7.26 (2H, m), 7.59 (1H, t, 8.0Hz), 7.72 (1H, d, J = 8.1 Hz), 7.85 (1 H, dd, J = 1.3Hz, 8.2Hz), 7.97 (1 H, dd, J = 1.3Hz, 7.5Hz), 8.66 (1 H, d, J = 2.2Hz), 9.27 (1H, d, J = 2.2Hz).
Mass Spectrum: C₁₇H₁₃ClN₂O₂S requires 344, 346; found 345, 347 (MH⁺)

### Description 7

### 8-Chloro-3-[(5-fluoro-2,3-dihydro-1H-isoindol-2-yl)sulfonyl]quinoline (D7)

To a stirred solution of 8-chloro-3-quinolinesulfonyl chloride (D5)(0.63 g, 2.4 mmol) in dichloromethane (10 ml) was added 2,3-dihydro-1*H*-isoindole hydrochloride (0.46 g, 2.6 mmol) and triethylamine (0.74 ml, 5.3 mmol) at 0 - 5°C under argon. After 1 h at this temperature range, the mixture was warmed to ambient temperature and stirred for 18 h. The precipitated solid was filtered off and purified by chromatography over silica gel eluting with a gradient of ethyl acetate/hexane to afford the title compound (D7) as a solid (0.48 g, 1.3 mmol, 54%).
Mass Spectrum: C₁₇H₁₂ClFN₂O₂S requires 362, 364; found 363, 365 (MH⁺)

### Description 8

### 8-Chloro-3-(1-piperidinylsulfonyl)quinoline (D8)

To a stirred solution of 8-chloro-3-quinolinesulfonyl chloride (D5)(0.4 g, 1.5 mmol) in dichloromethane (10 ml) was added piperidine (0.45 ml, 4.6 mmol) at 0 - 5°C under argon. After 1 h at this temperature range, the mixture was warmed to ambient temperature and stirred for 18 h. The solution was washed with water (2 x 15 ml), dried (MgSO₄) and concentrated *in vacuo* to give the title compound (D8) as a solid (0.297g, 1.0 mmol, 64%).
Mass Spectrum: C₁₄H₁₅ClN₂O₂S requires 310, 312; found 311, 313 (MH⁺)

### Description 9

### 8-Chloro-3-(4-morpholinylsulfonyl)quinoline (D9)

The title compound (D9) was prepared in 53% yield by a similar method to that of Description 8 using morpholine in place of piperidine.
Mass Spectrum: C₁₃H₁₃ClN₂O₃S requires 312, 314; found 313, 315 (MH⁺)

### Description 10

### 1,1-Dimethylethyl 4-[3-(2,3-dihydro-1H-indol-1-ylsulfonyl)-8-quinolinyl]-1-piperazinecarboxylate (D10)

A stirred suspension of 8-chloro-3-(2,3-dihydro-1*H*-indol-1-ylsulfonyl)quinoline (D6)(0.26 g, 0.74 mmol), 1,1-dimethylethyl 1-piperazinecarboxylate (0.152g, 0.81 mmol), tris(dibenzylideneacetone)dipalladium (0) (0.02 g, 0.022 mmol), 2-dicyclohexylphosphino-2'-(*N,N*-dimethylamino)biphenyl (0.026 g, 0.067 mmol) and sodium *tert*-butoxide (0.1 g, 1 mmol) in degassed 1,4-dioxan (3 ml) under argon was heated in a microwave oven in a sealed vessel to 125 °C for 0.25 h. The cooled reaction mixture was filtered and the filtrate concentrated *in vacuo* to a residue, which was purified by chromatography over silica gel eluting with a gradient of acetone/toluene to give the title compound (D10) as an oil (0.188 g, 038 mmol, 51%).
δ_{H} (CDCl₃) 1.49 (9H, s), 2.93 (2H, t, J = 8.5Hz), 3.28-3.32 (4H, m), 3.70-3.74 (4H, m), 4.01 (2H, t, J = 8.6Hz),6.94-7.28 (4H, m), 7.49-7.58 (2H, m), 7.72 (1H, d, J = 8.2Hz), 8.57 (1 H, d, J = 2.3Hz), 9.15 (1H, d, J = 2.3Hz).
Mass Spectrum: C₂₆H₃₀N₄O₄S requires 494; found 495 (MH⁺)

### Description 11-13 (D11-D13)

Description 11-13 (D11-D13) were prepared as described in Description 10 using the respective intermediates (D7), (D8) and (D9).

| **Compound** | **Mol Form.** **(requires)** | **MS** **(MH⁺)** |
|---|---|---|
| **1,1-Dimethylethyl4-{3-[(5-fluoro-2,3-dihydro-1*H*-isoindol-2-yl)sulfonyl]-8-quinolinyl}-1-piperazinecarboxylate (D11)** | C₂₆H₂₉FN₄O₄S (512) | 513 |
| **1,1-Dimethylethyl 4-[3-(1-piperidinylsulfonyl)-8-quinolinyl]-1-piperazinecarboxylate (D12)** | C₂₃H₃₂N₄O₄S (460) | 461 |
| **1,1-Dimethylethyl 4-[3-(4-morpholinylsulfonyl)-8-quinolinyl]-1-piperazinecarboxylate (D13)** | C₂₂H₃₀N₄O₅S (462) | 463 |

### Example 1

### 3-(2,3-Dihydro-1H-indol-1-ylsulfonyl)-8-(1-piperazinyl)quinoline hydrochloride (E1)

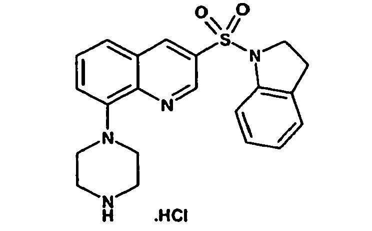

A stirred solution of 1,1-dimethylethyl 4-[3-(2,3-dihydro-1*H*-indol-1-ylsulfonyl)-8-quinolinyl]-1-piperazinecarboxylate (D10) (0.188 g, 0.38 mmol) in 1,4-dioxan (3 ml) and aqueous 4 M hydrochloric acid (3 ml) was heated at 80°C for 1.5 h under argon. The reaction mixture was concentrated *in vacuo* and the residue was stirred with diethyl ether (5 ml) and the resulting solid collected by filtration to afford the title compound (E1) 0.122 g, 0.28 mmol, 74%).
δ_{H} (d₆-DMSO) 2.89-2.96 (2H, m), 3.30 (4H, br, s), 3.54 (4H, br, s), 4.03-4.09 (2H, m), 6.99 (1H, dd, J = 0.9Hz, 7.4Hz), 7.14-7.26 (2H, m), 7.40 (1H, d, J = 6.7Hz), 7.57-7.69 (2H, m), 7.84 (1H, d, J = 7.3Hz), 8.98 (1H, d, J = 2.4Hz), 9.06 (1H, d, J = 2.4Hz), 9.30 (2H, br, s).
Mass Spectrum: C₂₁H₂₂N₄O₂S requires 394; found 395 (MH⁺)

### Example 2

### 3-[(5-Fluoro-2,3-dihydro-1H-isoindol-2-yl)sulfonyl]-8-(1-piperazinyl)quinoline hydrochloride (E2)

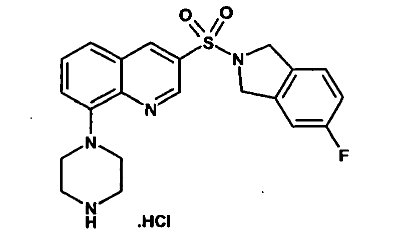

The title compound (E2) was prepared from 1,1-dimethylethyl 4-{3-[(5-fluoro-2,3-dihydro-1*H*-isoindol-2-yl)sulfonyl]-8-quinolinyl}-1-piperazinecarboxylate (D11) in a similar manner to that of Example 1, in 32% yield.
δ_{H} (d₆-DMSO) 3.34 (4H, br, s), 3.55 (4H, br, s), 4.67-4.70 (4H, m), 6.96-7.10 (2H, m), 7.23-7.28 (1H, m), 7.40 (1 H, d, J = 6.9Hz), 7.66 (1H, t, J = 8.0Hz), 7.85 (1 H, d, J = 7.8Hz), 8.96 (1H, d, J = 2.4Hz), 9.10 (2H, br, s), 9.17 (1H, d, J = 2.4Hz).
Mass Spectrum: C₂₁H₂₁ FN₄O₂S requires 412; found 413 (MH⁺)

### Example 3

### 8-(1-Piperazinyl)-3-(1-piperidinylsulfonyl)quinoline hydrochloride (E3)

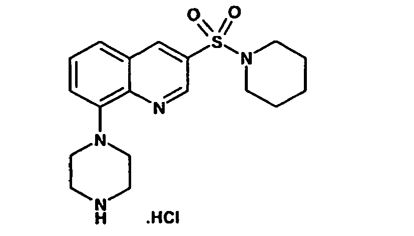

The title compound (E3) was prepared from 1,1-dimethylethyl 4-[3-(1-piperidinylsulfonyl)-8-quinolinyl]-1-piperazinecarboxylate (D12) in a similar manner to that of Example 1, in 77% yield.
δ_{H} (d₆-DMSO) 1.38 (2H, br, s), 1.56 (4H, br, s), 2.99-3.04 (4H, m), 3.35 (4H, br, s), 3.60 (4H, br, s), 7.42 (1H, d, J = 6.8Hz), 7.68 (1H, t, J = 8.0Hz), 7.87 (1H, d, J = 7.4Hz), 8.84 (1H, d, J = 2.4Hz), 9.05 (1 H, d, J = 2.4Hz), 9.40 (2H, br, s).
Mass Spectrum: C₁₈H₂₄N₄O₂S requires 360; found 361 (MH⁺)

### Example 4

### 3-(Morpholin-4-ylsulfonyl)-8-(1-piperazinyl)quinoline hydrochloride (E4)

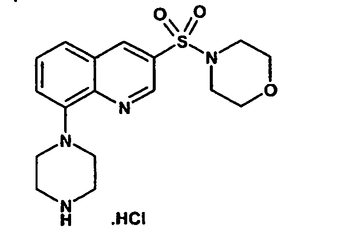

The title compound (E4) was prepared from 1,1-dimethylethyl 4-[3-(4-morpholinylsulfonyl)-8-quinolinyl]-1-piperazinecarboxylate (D13) in a similar manner to that of Example 1, in 26% yield.
δ_{H} (d₆-DMSO) 3.01 (4H, br, s), 3.40 (4H, br, s), 3.57-3.67 (8H, m), 7.44 (1 H, d, J = 6.6Hz), 7.70 (1H, t, J = 7.8Hz), 7.88 (1H, d, J = 7.3Hz), 8.87 (1 H, d, J = 2.4Hz), 9.06 (1H, d, J = 2.4Hz), 9.17 (2H, br, s).
Mass Spectrum: C₁₇H₂₂N₄O₃S requires 362; found 363 (MH⁺)

### Example 5

### 3-(2,3-Dihydro-1H-indol-1-ylsulfonyl)-8-(4-methyl-1-piperazinyl)quinoline hydrochloride (E5)

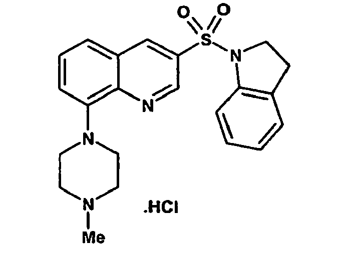

3-(2,3-Dihydro-1*H*-indol-1-ylsulfonyl)-8-(1-piperazinyl)quinoline hydrochloride (E1) (0.080 g, 0.19 mmol) was first converted to the corresponding free base by dissolving in a 5% aqueous solution of sodium hydrogen carbonate (5 ml) and extracting the solution with dichloromethane (3 x 5 ml). The combined extracts were dried (MgSO₄) and concentrated *in vacuo* to an oil which was identified as the free base of (E1).
A 37% aqueous solution of formaldehyde (0.030 ml, 0.4 mmol) was added to a stirred solution of 3-(2,3-dihydro-1*H*-indol-1-ylsulfonyl)-8-(1-piperazinyl)quinoline (E1) (0.056 g, 0.14 mmol) in ethanol (5 ml) and acetic acid (0.1 ml) at ambient temperature. Sodium triacetoxyborohydride (0.11 g, 0.52 mmol) was added and the mixture was stirred under argon for 0.4 h. The reaction mixture was passed down an SCX chromatography column eluting with a solution made up from concentrated aqueous ammonium hydroxide (specific gravity 0.88) and methanol (1:10 ^{vol}/ᵥₒₗ). Column fractions containing only the required product were pooled and concentrated *in vacuo* to give an oil which was dissolved in methanol and treated with a 1 M solution of hydrogen chloride in diethyl ether (1.1 molar equivalents). The solution was concentrated *in vacuo* and the residue was stirred with diethyl ether (2 ml) to give the title compound (E5) as a yellow solid (0.050 g, 0.12 mmol, 87%).
δ_{H} (d₆-DMSO) 2.85 (3H, d, J = 4.6Hz), 2.89-2.96 (2H, m), 3.14-3.40 (4H, m), 3.51-3.56 (2H, m), 3.96-4.13 (4H, m), 6.99 (1 H, dd, J = 1Hz, 7.4Hz), 7.14 (1H, d, J = 6.5Hz), 7.23 (1H, t, J = 7.3Hz), 7.40 (1H, d, J = 6.6Hz), 7.57-7.69 (2H, m), 7.84 (1H, d, J = 7.3Hz), 9.0 (1 H, d, J = 2.4Hz), 9.05 (1H, d, J = 2.4Hz), 10.6 (1 H, Br, s).
Mass Spectrum: C₂₂H₂₄N₄O₂S requires 408; found 409 (MH⁺)

### Pharmacological data

Compounds can be tested following the procedures outlined in WO98/27081.
The compounds of Examples E1-E5 were tested and showed good affinity for the 5-HT₆ receptor, having pKi values ≥ 7.5 at human cloned 5-HT₆ receptors.

## Claims

1. A compound of formula (I) or a pharmaceutically acceptable salt thereof: 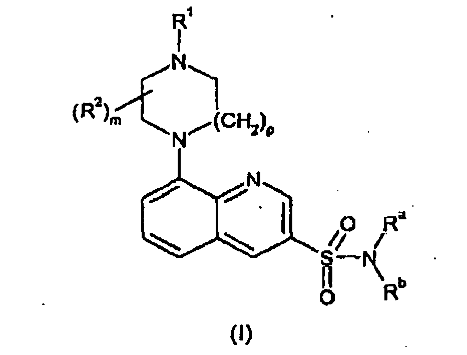 wherein:
R¹ represents hydrogen or methyl;
R² represents hydrogen or C₁₋₆ alkyl;
m represents an integer from 1 to 4, such that when m is an integer greater than 1, two R² groups may instead be linked to form a CH₂, (CH₂)₂ or (CH₂)₃ group;
when R¹ represents methyl, R¹ may optionally be linked to R² to form a group (CH₂)₂, (CH₂)₃ or (CH₂)₄;
p represents 1 or 2;
R**^{a}** and R^{b} together with the nitrogen atom to which they are attached represent a nitrogen containing Heterocyclyl group optionally substituted by a halogen atom; wherein said nitrogen containing heterocyclyl represents a 4-7 membered monocyclic saturated or partially unsaturated aliphatic ring or a 4-7 membered monocyclic saturated or partially unsaturated aliphatic ring containing 1 to 3 heteroatoms selected from oxygen and nitrogen fused to a benzene or monocyclic heteroaryl ring; or solvates thereof.

2. A compound of formula (I) as defined in claim 1, or a pharmaceutically acceptable salt thereof, wherein R² represents hydrogen, methyl or is linked to R¹ to form a (CH₂)₃ group.

3. A compound of formula (I) as defined in claim 1 which is:
3-(2,3-Dihydro-1*H*-indol-1-ylsulfonyl)-8-(1-piperazinyl)quinoline;
3-[(5-Fluoro-2,3-dihydro-1*H*-isoindol-2-yl)sulfonyl]-8-(1-piperazinyl)quinoline;
8-(1-Piperazinyl)-3-(1-piperidinylsulfonyl)quinoline;
3-(Morpholin-4-ylsulfonyl)-8-(1-piperazinyl)quinoline; or
3-(2,3-Dihydro-1*H*-indol-1-ylsulfonyl)-8-(4-methyl-1-piperazinyl)quinoline;
or a pharmaceutically acceptable salt thereof.

4. A pharmaceutical composition which comprises a compound as defined in any of claims 1 to 3 and a pharmaceutically acceptable carrier or excipient.

5. A compound as defined in any of claims 1 to 3 for use in therapy.

6. The use of a compound of formula (I) as defined in any of claims 1 to 3 or a pharmaceutically acceptable salt thereof in the manufacture of a medicament for the treatment or prophylaxis of depression, anxiety, Alzheimers disease, age related cognitive decline, ADHD, obesity, mild cognitive impairment, schizophrenia, cognitive deficits in schizophrenia and stroke.

## Patentansprüche

1. Verbindung der Formel (I) oder ein pharmazeutisch verträgliches Salz davon: 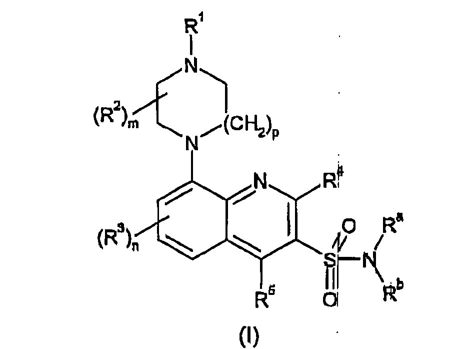 wobei:
R¹ ein Wasserstoffatom oder Methyl darstellt;
R² ein Wasserstoffatom oder C₁₋₆-Alkyl darstellt;
m eine ganze Zahl von 1 bis 4 darstellt, so däss, wenn m eine ganze Zahl größer als 1 ist, zwei Reste R² stattdessen verknüpft sein können, um eine CH₂-, (CH₂)₂- oder (CH₂)₃-Gruppe zu bilden;
wenn R¹ Methyl darstellt, kann R¹ gegebenenfalls mit R² verknüpft sein, um eine (CH₂)₂- (CH₂)₃- oder (CH₂)₄-Gruppe zu bilden;
p 1 oder 2 darstellt;
R^{a} und R^{b} zusammen mit den Stickstoffatom, an welches sie gebunden sind, einen Stickstoff enthaltenden heterocyclischen Rest darstellen, gegebenenfalls substituiert durch ein Halogenatom;
wobei der Stickstoff enthaltende Heterocyclus einen 4-7-gliedrigen monocyclischen gesättigten oder teilweise ungesättigten aliphatischen Ring oder einen 4-7-gliedrigen monocyclischen gesättigten oder teilweise ungesättigten aliphatischen ring enthaltend 1 bis 3 Heteroatome ausgewählt aus Sauerstoff und Stickstoff darstellt, kondensiert an einen Benzol- oder monocylischen Heteroarylring;
oder Solvate davon.

2. Verbindung der Formel (I) wie in Anspruch 1 definiert oder ein pharmazeutisch verträgliches Salz davon, wobei R² ein Wasserstoffatom oder Methyl darstellt oder an R¹ geknüpft ist, um eine (CH₂)₃-Gruppe zu bilden.

3. Verbindung der Formel (I) wie in Anspruch 1 definiert, nämlich:
3-(2,3-Dihydro-1*H*-indol-1-ylsulfonyl)-8-(1-piperazinyl)chinolin;
3-[(5-Fluor-2,3-dihydro-1*H*-isoindol-2-yl)sulfohyl]-8-(1-piperazinyl)chinolin
8-(1-Piperazinyl)-3-(1-piperidinylsulfonyl)chinolin;
3-(Morpholin-4-ylsulfonyl)-8-(1-piperazinyl)chinolin; oder
3-(2,3-Dihydro-1*H*-indol-1-ylsulfonyl)-8-(4-methyl-1-piperazinyl)chinolin;
oder ein pharmazeutisch verträgliches Salz davon.

4. Arzneimittel, welches eine Verbindung wie in einem der Ansprüche 1 bis 3 definiert und einen pharmazeutisch verträglichen Träger oder Exzipienten umfasst.

5. Verbindung wie in einem der Ansprüche 1 bis 3 definiert zur Verwendung in der Therapie.

6. Verwendung einer Verbindung der Formel (I) wie in einem der Ansprüche 1 bis 3 definiert oder ein pharmazeutisch verträgliches Salz davon zur Herstellung eines Medikaments zur behandlung oder Vorbeugung von Depression, Angst, Alzheimer-Krankheit, altersbedingtem kognitivem Verfäll, ADHS, Fettsucht, milder kognitiver Störung, Schizophrenie, kognitiven Defiziten bei Schizophrenie und Schlaganfall.

## Revendications

1. Composé de formule (I) ou un de ses sels pharmaceutiquement acceptables : 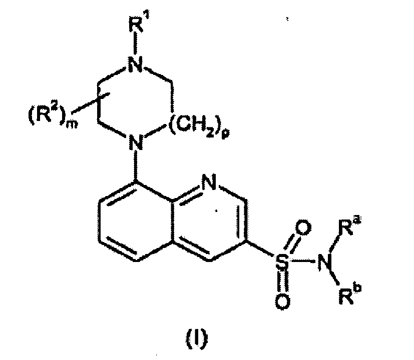 dans laquelle :
R¹ représente un atome d'hydrogène ou un groupe méthyle ;
R² représente un atome d'hydrogène ou un groupe alkyle en C₁ à C₆ ;
m représente un nombre entier de 1 à 4, de telle sorte que, lorsque m représente un nombre entier supérieur à 1, deux groupes R² puissent en variante être liés pour former un groupe CH₂, (CH₂)₂ ou (CH₂)₃;
lorsque R¹ représente un groupe méthyle, R¹ peut être éventuellement lié à R² pour former un groupe (CH₂)₂, (CH₂)₃ ou (CH₂)₄;
p est égal à 1 ou 2 ;
R^{a} et R^{b}, conjointement avec l'atome d'azote auquel ils sont fixés, représentent un groupe hétérocyclyle azoté facultativement substitué avec un atome d'halogène ;
ledit groupe hétérocyclyle azoté représentant un noyau aliphatique monocyclique tétra- à heptagonal saturé ou partiellement insaturé ou un noyau aliphatique tétra- à heptagonal monocyclique saturé ou partiellement insaturé contenant 1 à 3 hétéroatomes choisis entre l'oxygène et l'azote condensé avec un noyau benzénique ou noyau hétéroaryle monocyclique ;
ou ses produits de solvatation.

2. Composé de formule (I) suivant la revendication 1, ou un de ses sels pharmaceutiquement acceptables, dans lequel R² représente un atome d'hydrogène ou un groupe méthyle ou bien est lié à R¹ pour former un groupe (CH₂)₃.

3. Composé de formule (I) suivant la revendication 1, qui est :
la 3-(2,3-dihydro-1*H*-indole-1-ylsulfonyl)-8-(1-pipérazinyl)-quinoléine ;
la 3-[(5-fluoro-2,3-dihydro-1*H*-isoindole-2-yl)sulfonyl]-8-(1-pipérazinyl)-quinoléine ;
la 8-(1-pipérazinyl)-3-(1-pipéridinylsulfonyl)quinoléine ;
la 3-(morpholine-4-ylsulfonyl)-8-(1-pipérazinyl)-quinoléine ; ou
la 3-(2,3-dihydro-1*H*-indole-1-ylsulfonyl)-8-(4-méthyl-1-pipérazinyl)quinoléine ;
ou un de ses sels pharmaceutiquement acceptables.

4. Composition pharmaceutique qui comprend un composé tel que défini dans l'une quelconque des revendications 1 à 3 et un support ou excipient pharmaceutiquement acceptable.

5. Composé suivant l'une quelconque des revendications 1 à 3, destiné à être utilisé en thérapie.

6. Utilisation d'un composé de formule (I) suivant l'une quelconque des revendications 1 à 3 ou d'un de ses sels pharmaceutiquement acceptables dans la production d'un médicament destiné au traitement ou à la prophylaxie de la dépression, de l'anxiété, de la maladie d'Alzheimer, du déclin cognitif dû à l'âge, de la ADHD, de l'obésité, de l'altération cognitive bénigne, de la schizophrénie, des déficits cognitifs dans le cas de la schizophrénie et d'un ictus.
